# EUROPEAN PATENT SPECIFICATION

(11) **EP 4 250 314 B1**
(45) Date of publication and mention of the grant of the patent: **11.02.2026**
(21) Application number: 23186280.6
(22) Date of filing: 02.08.2019
(51) Int. Cl.: A61C 17/22, A46B 11/00, A46B 15/00, G16H 10/00, G16H 80/00

(54) **SYSTEM FOR DETECTING BLOOD IN AN ORAL CAVITY DURING TOOTHBRUSHING**
SYSTEM ZUM NACHWEIS VON BLUT IN EINER MUNDHÖHLE WÄHREND DES ZAHNBÜRSTENS
SYSTÈME DE DÉTECTION DE SANG DANS UNE CAVITÉ BUCCALE PENDANT LE BROSSAGE DES DENTS

(43) Date of publication of application: 27.09.2023
(62) Divisional of application: 19755470.2
(73) Proprietor: Colgate-Palmolive Company, New York, NY 10022 (US)
(72) Inventor: WU, Donghui, Bridgewater 08807 (US); FITZGERALD, Michael, Oakhurst 07755 (US)
(74) Representative: Mooser, Sebastian Thomas

(56) References cited:
- WO-A1-2018/065374
- WO-A1-2020/161604
- US-A1- 2014 323 836
- US-A1- 2017 020 277

## Description

### BACKGROUND

There are several different causes for gum and other oral cavity bleeding. Some of them are mechanical issues, such as excessive brushing force, using a toothbrush with stiff bristles, incorrect brushing or flossing mechanics, or improperly fitted dentures. Gum disease can also cause bleeding due to inflammation of gum tissues. Other important causes can be systemic, such as medications (e.g. blood thinners), pregnancy (hormonal changes), diabetes, vitamin deficiency (e.g. C or K), Leukemia, etc. Although the American Dental Association recommends that people with gum bleeding see a dentist or physician, a majority of the population do not feel pain when they have bleeding gums and therefore ignore this recommendation. Therefore, there is a need to provide consumers with a toothbrushing system that can monitor and/or log gum bleeding during routine tooth brushing.

Prior art is found in US 2014/323836 which generally relates to a biological information acquisition terminal, information management method, and information display method, and in WO 2018/065374 A1 which generally relates to a smart toothbrush.

### BRIEF SUMMARY

The invention is set out in the independent claims. Preferred embodiments of the invention are set out in the dependent claims.

The invention is directed to a system for detecting hemoglobin and/or blood in the oral cavity during toothbrushing, as well as to a toothbrush capable of doing the same.

According to a first aspect, we describe a system for detecting blood in an oral cavity during toothbrushing, the system comprising: a toothbrush comprising an electronic circuit comprising a sensor that is configured to generate signals related to the presence or absence of hemoglobin in the oral cavity during a toothbrushing session; and a portable electronic device comprising a processor that is operably coupled to the sensor of the toothbrush, the processor configured to receive and process the signals to determine whether hemoglobin is present in the oral cavity during the toothbrushing session, wherein the sensor is configured to emit first light at a first wavelength and second light at a second wavelength, receive reflected portions of the first light and the second light, and generate a first signal indicative of a first intensity of the reflected portion of the first light and a second signal indicative of a second intensity of the reflected portion of the second light; wherein the processor is configured to calculate a ratio of the first intensity to the second intensity to determine whether hemoglobin is present in the oral cavity during the toothbrushing session; and wherein the first light is red light or infrared light and the second light is green light.

According to a second aspect, we describe a system for detecting blood in a toothpaste slurry during a toothbrushing session, the system comprising: a sensor configured to generate signals related to the presence or absence of hemoglobin in the toothpaste slurry during the toothbrushing session; a processor operably coupled to the sensor and configured to receive and process the signals to determine whether hemoglobin is present in the toothpaste slurry during the toothbrushing session; and an output configured to inform a user whether blood is present in the toothpaste slurry during the toothbrushing session based on the processing performed by the processor, wherein the sensor is configured to emit first light at a first wavelength and second light at a second wavelength, receive reflected portions of the first light and the second light that are reflected from the toothpaste slurry, and generate a first signal indicative of a first intensity of the reflected portion of the first light and a second signal indicative of a second intensity of the reflected portion of the second light, and wherein the processor is configured to calculate a ratio of the first intensity to the second intensity to determine whether hemoglobin is present in the toothpaste slurry.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:
FIG. 1 is a perspective view of a toothbrush having a body and a refill head in accordance with an embodiment of the present invention, with the refill head in a detached state;
FIG. 2 is a perspective view of the toothbrush of FIG. 1 with the refill head in an attached state;
FIG. 3 is a cross-sectional view taken along line III-III of FIG. 2;
FIG. 4 is a schematic illustration of a sensor of the toothbrush of FIG. 1;
FIGS. 5A and 5B are schematic illustrations of the sensor of FIG. 4 transmitting light into a toothpaste slurry and receiving reflected light in accordance with embodiments of the present invention;
FIG. 6 is a scatterplot illustrating the results of calculations performed by the toothbrush of FIG. 1 to determine the presence of hemoglobin in the oral cavity in accordance with an experiment;
FIG. 7 is a graph illustrating the experimental results of calculations performed by the toothbrush of FIG. 1 to determine the presence of hemoglobin in the oral cavity;
FIG. 8 illustrates a system for detecting blood in an oral cavity that includes a toothbrush and a portable electronic device that are in operable communication with one another;
FIG. 9 is an electrical block diagram of the electronic components of the toothbrush and the portable electronic device of the system of FIG. 8;
FIGS. 10A and 10B are illustrations of a software application launched on a display device of the portable electronic device of the system of FIG. 8, wherein the software application is indicating whether or not blood was found in the oral cavity;
FIG. 11 is an illustration of a software application launched on a display device of the portable electronic device of the system of FIG. 8, wherein the software application is displaying a log of data related to the detection of blood in the oral cavity during numerous toothbrushing sessions;

### DETAILED DESCRIPTION

The following description of the preferred embodiment(s) is merely exemplary in nature and is in no way intended to limit the invention, its application, or uses.

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention. Relative terms such as "lower," "upper," "horizontal," "vertical," "above," "below," "up," "down," "top" and "bottom" as well as derivatives thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the invention are illustrated by reference to the exemplified embodiments. Accordingly, the invention expressly should not be limited to such exemplary embodiments illustrating some possible non-limiting combination of features that may exist alone or in other combinations of features; the scope of the invention being defined by the claims appended hereto.

As used throughout, ranges are used as shorthand for describing each and every value that is within the range. Any value within the range can be selected as the terminus of the range.

Features of the present invention may be implemented in software, hardware, firmware, or combinations thereof. The computer or software programs described herein are not limited to any particular embodiment, and may be implemented in an operating system, application program, foreground or background processes, driver, or any combination thereof. The computer programs may be executed on a single computer or server processor or multiple computer or server processors.

Processors described herein may be any central processing unit (CPU), microprocessor, micro-controller, computational, or programmable device or circuit configured for executing computer program instructions (e.g. code). Various processors may be embodied in computer and/or server hardware of any suitable type (e.g. desktop, laptop, notebook, tablets, cellular phones, etc.) and may include all the usual ancillary components necessary to form a functional data processing device including without limitation a bus, software and data storage such as volatile and non-volatile memory, input/output devices, graphical user interfaces (GUIs), removable data storage, and wired and/or wireless communication interface devices including Wi-Fi, Bluetooth, LAN, etc.

Computer-executable instructions or programs (e.g. software or code) and data described herein may be programmed into and tangibly embodied in a non-transitory computer-readable medium that is accessible to and retrievable by a respective processor as described herein which configures and directs the processor to perform the desired functions and processes by executing the instructions encoded in the medium. It should be noted that non-transitory "computer-readable medium" as described herein may include, without limitation, any suitable volatile or non-volatile memory including random access memory (RAM) and various types thereof, read-only memory (ROM) and various types thereof, USB flash memory, and magnetic or optical data storage devices (e.g. internal/external hard disks, floppy discs, magnetic tape CD-ROM, DVD-ROM, optical disk, ZIP^{™} drive, Blu-ray disk, and others), which may be written to and/or read by a processor operably connected to the medium.

In certain embodiments, the present invention may be embodied in the form of computer-implemented processes and apparatuses such as processor-based data processing and communication systems or computer systems for practicing those processes. The present invention may also be embodied in the form of software or computer program code embodied in a non-transitory computer-readable storage medium, which when loaded into and executed by the data processing and communications systems or computer systems, the computer program code segments configure the processor to create specific logic circuits configured for implementing the processes.

The invention described herein relates to an apparatus (i.e., toothbrush), system for detecting blood in an oral cavity during toothbrushing and a system for for detecting blood in a toothpaste slurry during a toothbrushing session using sensors that measure the hemoglobin level in saliva/toothpaste slurry. In one aspect, the detection of gum or other soft tissue bleeding occurs during toothbrushing, so the sensors may be described herein as being located on a toothbrush. The sensor may include a light transmitter and a light receiver. The light transmitter may emit visible and infrared light during toothbrushing and the intensity of the reflected light (i.e., the light reflected back from the toothpaste slurry) is received by the light receiver. Because hemoglobin has a strong red color, it absorbs green light while reflecting the majority of the red and infrared light back. Therefore, using a ratio of reflected light intensity (red/green and/or infrared/green) and applying that data to a processing algorithm, the hemoglobin can be quantified. The information obtained can either be stored on a memory device in the toothbrush or automatically transferred to a mobile phone (or other portable electronic device) app (software application), or both. In either case, the information can be provided to a user (either as logs of all information or as an indicator that blood was present in the toothpaste slurry) so that a user can be informed about gum bleeding.

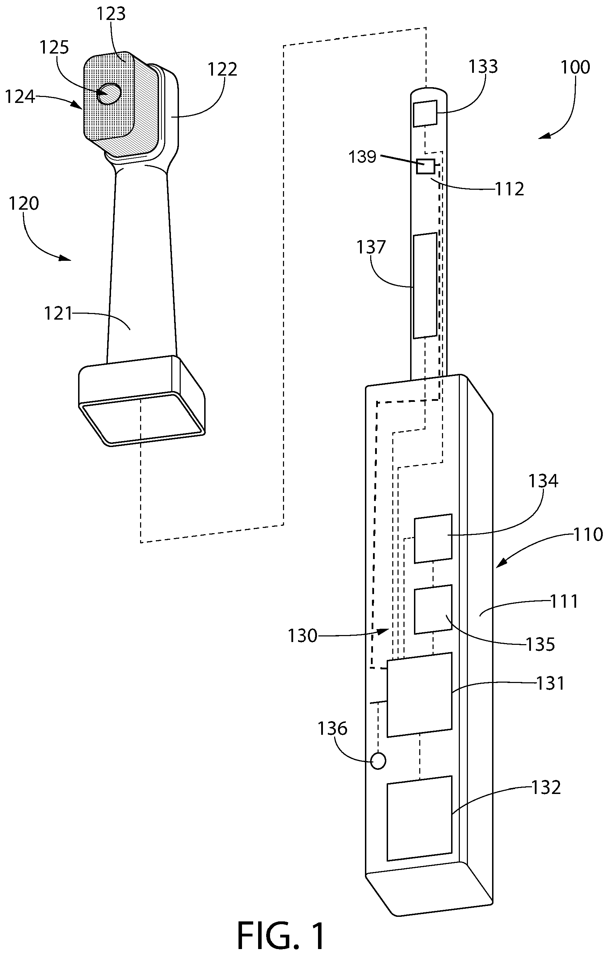

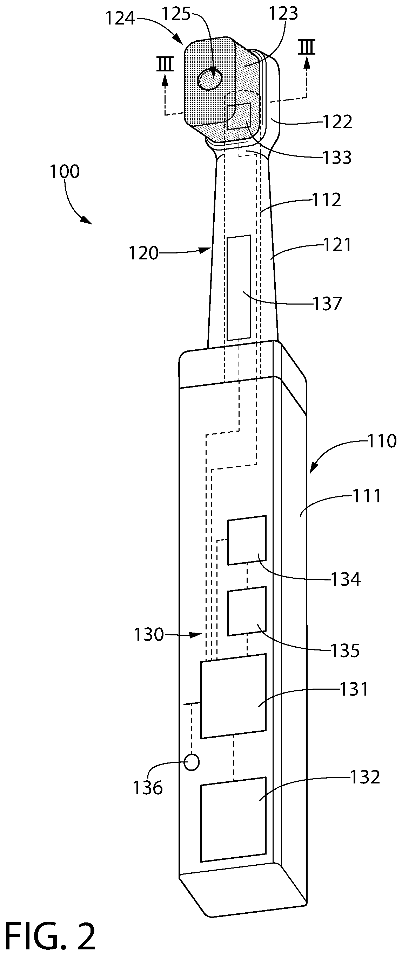

Referring to FIGS. 1 and 2, a toothbrush 100 is illustrated in accordance with an embodiment of the present invention. In the exemplified embodiment, the toothbrush 100 generally comprises a body 110 and a refill head 120 that is detachably coupled to the body 110. More specifically, the body 110 comprises a handle portion 111 that is configured for gripping and handling by a user and a stem 112 that is configured for attachment of the refill head 120 to the body 110. The refill head 120 comprises a sleeve portion 121 and a head portion 122. The sleeve portion 121 is sized and configured to fit over the stem 112 of the body 110 for coupling the refill head 120 to the body 110. The refill head 120 may be coupled to the body 110 with a friction/interference fit or via mechanical interaction, such as the refill head 120 having a protuberance or recess that matches with a recess or protuberance on the body 110. Various techniques for coupling a refill head 120 to a body 110 of a toothbrush 100 are known and could be used in accordance with the invention described herein (i.e., magnetic, mechanical, interference, screw threads, protuberance/detent, or the like). The refill head 120 and the body 110 are illustrated generically and the invention is not to be limited by the shape, size, and/or geometry of these components.

The refill head 120 also comprises cleaning elements 123 that extend from the head portion 122. The cleaning elements 123 may be bristles, elastomeric fingers, lamella, rubber elements, or the like. Specifically, the cleaning elements 123 may be any feature or structure that is known to be used for cleaning of the teeth, gums, and other oral cavity surfaces. The pattern, material, shape, rigidity, or the like of the cleaning elements is not to be limiting of the invention.

In certain embodiments, the exact structure, pattern, orientation, and material of the tooth cleaning elements 123 are not to be limiting of the present invention. Thus, the term "tooth cleaning elements" may be used herein in a generic sense to refer to any structure that can be used to clean, polish or wipe the teeth and/or soft oral tissue (e.g. tongue, cheek, gums, etc.) through relative surface contact. Common examples of "tooth cleaning elements" include, without limitation, bristle tufts, filament bristles, fiber bristles, nylon bristles, spiral bristles, rubber bristles, elastomeric protrusions, flexible polymer protrusions, combinations thereof, and/or structures containing such materials or combinations. Suitable elastomeric materials include any biocompatible resilient material suitable for uses in an oral hygiene apparatus. To provide optimum comfort as well as cleaning benefits, the elastomeric material of the tooth or soft tissue engaging elements has a hardness property in the range of A8 to A25 Shore hardness. One suitable elastomeric material is styrene-ethylene/butylene-styrene block copolymer (SEBS) manufactured by GLS Corporation. Nevertheless, SEBS material from other manufacturers or other materials within and outside the noted hardness range could be used.

The tooth cleaning elements 123 of the present invention can be connected to the head portion 122 of the refill head 120 in any manner known in the art. For example, staples/anchors, in-mold tufting (IMT), anchor free tufting (AFT), PTt anchorless tufting, or the like could be used to mount the cleaning elements/tooth engaging elements to the head portion 122 of the refill head 120.

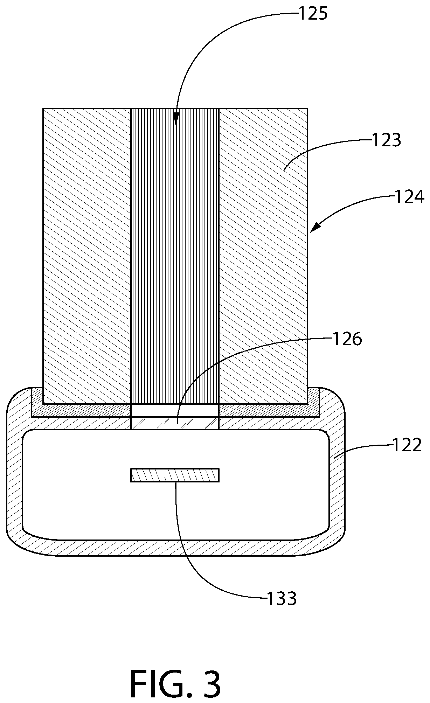

In the exemplified embodiment, the cleaning elements 123 define a cleaning element field 124. Furthermore, the cleaning element field 124 defines an opening or cavity 125, which is formed by a portion of the head portion 122 not having any cleaning elements 123 extending therefrom. Thus, there is a portion of the head portion 122 that is devoid of any cleaning elements 123, and this portion is surrounded by the cleaning elements 123 to form the cavity 125 within the cleaning element field 124. Of course, in other embodiments the spacing between the cleaning elements 123 of the cleaning element field 124 may be different than that which is shown such that there is no cavity per se, but still so that a gap in the cleaning element field 124 remains. The purpose of this opening or cavity 125 (or gap in the cleaning element field 124) will be better understood from the description below with particular reference to FIG. 3.

Although in the exemplified embodiment the toothbrush 100 is one which includes a body 110 and a refill head 120 that are detachably coupled together, in other embodiments the toothbrush 100 may be a unitary component comprising a handle and a head that are fixedly coupled together, such as with most traditional manual toothbrushes in the market. Having a detachable refill head 120 may be desirable because it can prolong the use of the toothbrush 100 by enabling a user to replace the refill head 120 and hence also the bristles 123 as they become worn while reusing the body 120 which includes expensive circuitry and electronic components as described further herein below. However, it is possible to utilize the techniques and components described herein on a more conventional manual toothbrush that does not include a replaceable refill head. Thus, the invention is not limited to one which requires the toothbrush to include a refill head in all embodiments unless specifically claimed as such.

In the exemplified embodiment, the toothbrush 100 comprises an electronic circuit 130 for acquiring and/or generating signals related to detecting the presence of hemoglobin (and hence also blood) in the oral cavity (or a toothpaste slurry) during a toothbrushing session. The toothbrush 100 (and related system described below) is able to detect and measure hemoglobin/blood using a few wavelengths (two to three) in visible and/or infrared regions during toothbrushing. Thus, no reagents, assays, wet chemistry preparation, and/or specialized equipment is needed to detect the hemoglobin/blood using the techniques described herein.

As mentioned above, in the exemplified embodiment the components of the electronic circuit 130 are located within the body 110 of the toothbrush 100, which is a non-replaceable part of the toothbrush. In that regard, the body 110 of the toothbrush 100 comprises a cavity or otherwise hollow region within which the components of the electronic circuit 130 are located. Because the electronic circuit 130 is located within the body 110 and not the refill head 120, as the cleaning elements 123 on the refill head 120 become worn, a new refill head 120 can be attached to the body 110 to prolong the usable life of the toothbrush 100. This is one reason that forming the toothbrush 100 so that it includes a body 110 and a detachable refill head 120 may be desirable.

In the exemplified embodiment, the electronic circuit 130 comprises, in operable coupling, a processor 131, a power source 132, a sensor 133, a memory device 134 (which could alternatively be a part of the processor 131 in some embodiments), a Bluetooth module 135, and an indicator 136. The components of the electronic circuit 130 are all located within a cavity in the body 110. Furthermore, in the exemplified embodiment the electronic circuit 130 also comprises a motor 137 that is operably coupled to the processor 131 for imparting motion and/or vibrations to the refill head 120 in instances in which the toothbrush 100 is a powered toothbrush instead of a manual toothbrush. For example, the motor 137 could include an eccentric counterweight to provide vibration during tooth cleaning. The processor 131 may be considered a microcontroller in some embodiments because may include all peripherals in the chip itself and may not run on any operating system.

The illustration of FIGS. 1 and 2 is schematic in that it illustrates each of the components of the electronic circuit 130 with a box and uses a dashed line to illustrate the electrical coupling between the components. These boxes are not actually visible on the exterior of the body 110 but rather they are representative of the components of the electronic circuit 130 as described herein. Specifically, the boxes represent components of the electronic circuit 130 that are housed within a cavity defined by the body 110.

In the exemplified embodiment, the motor 137 and the sensor 133 are located in the stem 112 of the body 110 and the processor 131, the power source 132, the memory device 134, the Bluetooth module 135, and the indicator 136 are located in the handle portion 111 of the body 110. In some embodiments, the processor 131 may be located in the stem 112 alongside of the sensor 133 rather than in the handle portion 111. The sensor 133 may be connected to the processor 131 using any desired techniques, although a standard ADC, I²C or SPI interface may be used in some embodiments. In some embodiments, the Bluetooth module 135 may be coupled to the processor 131 through a standard serial interface. In other embodiments, the Bluetooth module 135 and the processor 131 can be combined as a single unit. Furthermore, as noted above the memory device 134 may be a part of the processor 131 in some embodiments, and in other embodiments the memory device 134 may be omitted entirely.

Referring to FIG. 3, when the refill head 120 is coupled to the body 110, the cavity 125 in the cleaning element field 124 is aligned with the sensor 133 of the electronic circuit 130. Furthermore, the head portion 120 may include an optical transparent window 126 that is aligned with the sensor 133 and the cavity 125 to prevent fluids such as saliva, water, and toothpaste slurry from contacting the sensor 133. It can be important to align the sensor 133 with the cavity 125 in the cleaning element field 124 because, as described in more detail below, in some embodiments the sensor 133 is configured to emit and receive light. Thus, in the exemplified embodiment there is a passageway through the cleaning element field 124 for the light to be passed from the sensor 133 into a user's oral cavity and then back from the oral cavity to the sensor 133 during toothbrushing. As noted above, in the exemplified embodiment this is achieved with the combination of the optical transparent window 126 and the cavity 125 in the cleaning element field 124.

In the exemplified embodiment, the sensor 133, and hence also the cavity 125, is located centrally along the bristle field. However, the invention is not to be so limited in all embodiments. In other embodiments, the sensor 133 may be located so as to be aligned with the proximal end of the head 120 with the bristle field 124 being located entirely between the sensor 133 and the distal end of the head 120. In such an embodiment, there may be no need for the bristle field 124 to include the cavity 124 because there will be no overlap between the bristle field 124 and the sensor 133.

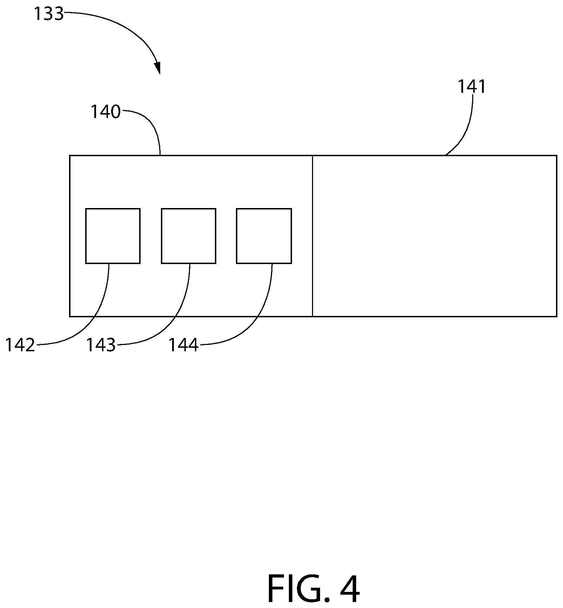

Referring to FIG. 4, the sensor 133 will be described in greater detail in accordance with an embodiment of the present invention. The sensor 133 is preferably small so that it can fit within the stem 112 and/or the head portion 122 of the toothbrush 100 as depicted in the figures. In the exemplified embodiment the sensor 133 comprises a transmitter 140 and a receiver 141. Furthermore, in the exemplified embodiment the transmitter 140 comprises a first light source 142, a second light source 143, and a third light source 144. Although three light sources are depicted in the exemplified embodiment, the invention is not to be so limited in all embodiments. Specifically, in some alternative embodiments the transmitter 140 may include only the first and second light sources 142, 143 and not also the third light source 144. One example of the sensor 133 is MAX30105 from Maxim Integrated, although the invention is not to be limited to this in all embodiments and other sensors could be used. In some embodiments, the transmitter 140 can be a broadband white light emitter. In such an embodiment, the receiver 141 may have multiple channels to detect reflected light at different wavelengths separately.

In the exemplified embodiment, the sensor 133 is a singular structure that includes all of the features/components noted herein. However, the invention is not to be so limited and in some other embodiments the sensor may comprise multiple sensors that are independent and distinct from one another. For example, the sensor may include discrete light sources that are separate and apart from a receiver. Thus, the term sensor, as used herein, includes the situation where a single sensor having all of the necessary components is used and the situation where multiple sensors that in combination have all of the necessary components are used.

The first light source 142 is configured to emit light at a first wavelength, the second light source 143 is configured to emit light at a second wavelength that is different than the first wavelength, and the third light source is configured to emit light at a third wavelength that is different than the first and second wavelengths. For example, in one embodiment the first light source 142 is configured to emit red light having a wavelength in a range of 625-740 nm, more specifically 640-680 nm. Furthermore, in one embodiment the second light source 143 is configured to emit green light having a wavelength in a range of 520-560 nm, more specifically 520-540 nm. Further still, in one embodiment the third light source 144 is configured to emit infrared light having a wavelength in a range of 700 nm - 1 micron, and more specifically 830-930 nm, and still more specifically 860-900 nm. In the exemplified embodiment, each of the first, second, and third light sources 142, 143, 144 are light emitting diodes, although other types of light sources may be used in the alternative. Thus, the transmitter 140 of the sensor 131 comprises multiple light sources such that each of the light sources transmits light at a different wavelength. The receiver 141 may be a broad spectrum light detector such that it can detect reflected light in all of the wavelengths mentioned herein (i.e., it can detect at least red, green, and infrared light).

In the exemplified embodiment the sensor 133 is operably coupled to the processor 131 so that measurements or other information detected by the sensor 133 can be transmitted to the processor 131 as a signal for processing. Furthermore, the processor 131 is pre-programmed with algorithms or otherwise works in association with software applications containing algorithms so that the processor 131 can perform various calculations using the information acquired by the sensor 133 to determine whether hemoglobin, and hence also blood, is being detected by the sensor 133. Specifically, the processor 131 can perform calculations and then, utilizing the pre-programmed algorithms, determine whether the results of those calculations indicate that hemoglobin/blood is present or not, and if so at what quantity.

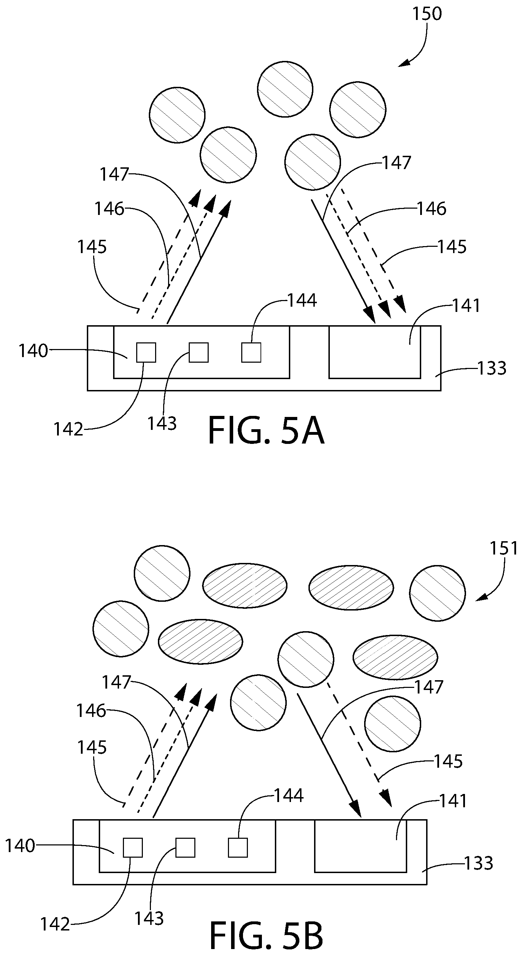

Specifically, referring to FIGS. 5A and 5B, operation of the sensor 133 will be described. FIG. 5A schematically depicts the sensor 133 located within an oral cavity so that light emitted by the sensor 133 can contact and be reflected by a toothpaste slurry 150 in the oral cavity that is devoid of any blood. FIG. 5B schematically depicts the sensor 133 located within an oral cavity so that the light emitted by the sensor 133 can contact and be reflected by a toothpaste slurry 151 in the oral cavity that comprises blood. A toothpaste slurry is a liquid formulation that includes toothpaste and saliva. Furthermore, if there is blood in the mouth, this blood will mix with the liquid formulation and also form a part of the toothpaste slurry. Thus, the sensor 133 is configured to detect whether there is blood in the toothpaste slurry, which would in turn be indicative of bleeding occurring within the oral cavity (i.e., gum bleeding or the like).

Referring first to FIG. 5A, the sensor 133 is located within an oral cavity that has a toothpaste slurry 150 therein that is devoid of any blood. Thus, the toothpaste slurry 150 includes toothpaste and saliva, but no blood. In this embodiment, the first light source 142 emits a first light 145 into the oral cavity and towards the toothpaste slurry 150 at the first wavelength (e.g., red light), the second light source 143 emits a second light 146 into the oral cavity and towards the toothpaste slurry 150 at the second wavelength (e.g., green light), and the third light source 144 emits a third light 147 into the oral cavity and towards the toothpaste slurry 150 at the third wavelength (e.g., infrared light). In a complicated matrix such as that of saliva/toothpaste slurry, abrasive particles and air bubbles in the toothpaste slurry reflect light at different wavelengths at a similar efficiency. Thus, as shown in FIG. 5A, the first, second, and third lights 145, 146, 147 are all reflected off of the toothpaste slurry 150 as a reflected portion of the first light 145, a reflected portion of the second light 146, and a reflected portion of the third light 147.

Referring to FIG. 5B, the sensor 133 is located within an oral cavity that has a toothpaste slurry 151 therein that comprises blood. Thus, the toothpaste slurry 151 includes toothpaste, saliva, and blood due to gum or other oral tissue surface bleeding within the oral cavity. In this embodiment, the first light source 142 emits the first light 145 into the oral cavity and towards the toothpaste slurry 151 at the first wavelength (e.g., red light), the second light source 143 emits a second light 146 into the oral cavity and towards the toothpaste slurry 151 at the second wavelength (e.g., green light), and the third light source 144 emits a third light 147 into the oral cavity and towards the toothpaste slurry 151 at the third wavelength (e.g., infrared light). Due to its strong red color, hemoglobin in red blood cells will strongly absorb green light, while reflecting a majority of red and infrared light back.

Thus, as shown in FIG. 5B, the first and third light 145, 147 are reflected from the toothpaste slurry 151 as a reflected portion of the first light 145 and a reflected portion of the third light 147. However, the second light 146 (which is the green light in the exemplified embodiment) is absorbed by the toothpaste slurry 151. In FIG. 5B, it is illustrated such that none of the second light 146 is reflected back to the receiver 142 of the sensor 133. However, in actual practice some of the second light 146 is reflected back, but it has a reduced intensity as compared to the amount of the second light 146 that is reflected back from the toothpaste slurry 150 of FIG. 5A that is devoid of blood due to the hemoglobin absorbing some of the second light 146. Thus, the second light 146 that is reflected from the toothpaste slurry 150 that is devoid of blood has a greater intensity than the second light 146 that is reflected from the toothpaste slurry 151 that comprises blood due to the hemoglobin in the blood absorbing some of the second light 146.

Thus, during toothbrushing with the cleaning elements 123 of the toothbrush 100, the sensor 133 transmits the first, second, and third lights 145, 146, 147 into the oral cavity. The first, second, and third lights 145, 146, 147 contact the toothpaste slurry 150, 151 in the oral cavity and reflected portions of the first, second, and third lights 145, 146, 147 are received by the receiver 141 of the sensor 133. The intensity of the reflected portions of the first and third lights 145, 147 (red and infrared light) are relatively unchanged regardless of whether or not the toothpaste slurry comprises blood. However, the intensity of the reflected portion of the second light 146 (green light) is greater when the toothpaste slurry does not comprise blood than when it does. Thus, the sensor 133 generates a first signal indicative of a first intensity of the reflected portion of the first light 145, a second signal indicative of a second intensity of the reflected portion of the second light 146, and a third signal indicative of the third intensity of the reflected portion of the third light 147.

Because the intensity of the reflected portion of the second light 146 is reduced when there is blood in the toothpaste slurry while the intensity of the reflected portions of the first and third light 145, 147 remain substantially the same regardless of whether or not there is blood in the toothpaste slurry, the ratio of the reflected light intensities can be used to identify and quantify hemoglobin/blood. Thus the processor 131 may have an algorithm that can calculate the ratios and determine whether or not (and how much) hemoglobin and blood is present.

Due to their operable coupling, the first, second, and third signals (although the third signal could be omitted because the system could operate just as well with only red or infrared light and green light being transmitted by the sensor 133) is transmitted from the sensor 133 to the processor 131. The processor 131 is equipped with algorithms instructing it to perform calculations with the first, second, and third signals to assist in determining whether hemoglobin/blood is in the toothpaste slurry. Specifically, the processor 131 is configured to calculate a ratio of the first intensity of the first light 145 to the second intensity of the second light 146 and/or a ratio of the third intensity of the third light 147 to the second intensity of the second light 146. As should be appreciated, if there is hemoglobin/blood in the toothpaste slurry, the intensity of the reflected portion of the second light 146 is less than if there is no hemoglobin in the toothpaste slurry. Thus, if there is hemoglobin/blood in the toothpaste slurry, the ratio of the first intensity of the first light 145 to the second intensity of the second light 146 and the ratio of the third intensity of the third light 147 to the second intensity of the second light 146 is increased as compared to the situation where there is no hemoglobin/blood in the toothpaste slurry (due to the denominator in the ratio calculation being reduced). Using this understanding and the developed algorithms, the processor 131 can make a determination as to whether there is hemoglobin/blood in the toothpaste slurry, and if so, how much.

For example, the processor 131 or algorithm may be set with a predetermined threshold for the various ratios that are calculated so that upon the ratio exceeding the predetermined threshold, the processor 131 will be informed that hemoglobin/blood has been detected. Thus, the processor 131 can be programmed so that if the ratio of the first intensity of the first light 145 to the second intensity of the second light 146 exceeds a first predetermined threshold and/or the ratio of the third intensity of the third light 147 to the second intensity of the second light 146 exceeds a second predetermined threshold, hemoglobin/blood is present.

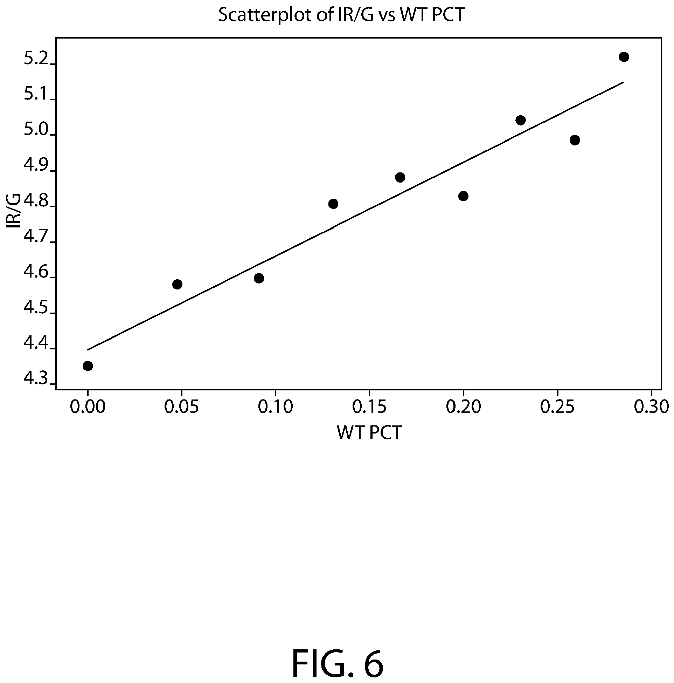

These predetermined thresholds may be determined based on testing with baseline samples of toothpaste slurry that do not include hemoglobin/blood and testing with test samples of toothpaste slurry that include varying amounts of toothpaste slurry, as discussed further below with reference to FIG. 6. Thus, the predetermined thresholds may be determined by running tests with the toothbrush 100 on toothpaste slurries that do not have any hemoglobin such that if the ratio is increased from the test data, it can be determined that there is blood present in the toothpaste slurry.

Specifically, FIG. 6 is a scatterplot illustrating the results of an experiment testing in-vitro detection of hemoglobin in blood by a prototype device having the same technology that is present in the toothbrush 100 described herein. In the experiment, lyophilized human hemoglobin (Sigma) was reconstituted in deionized water to a concentration of 4.2% and combined with a 1 part : 3 part w/w slurry of Colgate Dental Cream (Great Regular Flavor) and deionized water to form solutions of the following final w/w concentrations of hemoglobin:

| **Wt. Pct. Hemoglobin** |
|---|
| 0.285714 |
| 0.259259 |
| 0.230769 |
| 0.2 |
| 0.166667 |
| 0.130435 |
| 0.090909 |
| 0.047619 |

As should be appreciated, the solutions with a higher concentration of hemoglobin will have a darker red color, and therefore will absorb more green light that is transmitted at it. A single 50 microliter droplet of each solution was measured using the prototype device. FIG. 6 is a scatterplot indicating a linear relationship between the measured values of reflected infrared and green light (expressed as the ratio IR/G) and the concentration of hemoglobin. As can be seen, as the wt. % of hemoglobin increases, the results of the ratio IR/G also increases (and the same occurs if the infrared light is replaced with red light). This is because with more hemoglobin present in the solution, more of the green light is absorbed by the solution and the reflected green light has a lower intensity. Thus, using this information, an algorithm can be created to determine the presence or lack thereof of hemoglobin in a toothpaste slurry and also the quantity of the hemoglobin (and therefore also blood) in the toothpaste slurry. In this example, if the ratio of IR/G is greater than approximately 4.5, it is likely that there is blood in the solution being tested (i.e., the toothpaste slurry). Thus, the predetermined threshold could be 4.5 when the light sources emit infrared and green light, respectively, in one embodiment.

Referring again to FIGS. 1 and 2, as noted above the electronic circuit 130 also comprises the indicator 136. In the exemplified embodiment, the indicator 136 is located on the body 110 of the toothbrush 100. The indicator 136 is operably coupled to the processor 131 so that upon the processor 131 determining that there is blood in the oral cavity or the toothpaste slurry, the processor 131 can activate the indicator 136. In one embodiment, the indicator 136 may be a light, such as a light emitting diode (LED) or the like. In such an embodiment, upon the processor 131 determining that there is hemoglobin/blood in the oral cavity (based on one of the ratios noted above being calculated to be above its corresponding predetermined threshold or using some other determination as set out in an algorithm), the processor 131 will activate the indicator 136 so that it lights up/illuminates, thereby indicating to the user that there is blood in the oral cavity (i.e. that the user's gums or the like are bleeding). Of course, the indicator 136 is not limited to being a light source in all embodiments, and it could be an audio source in other embodiments such that when activated it emits a sound that is audible to the user. In still other embodiments, the indicator 136 could take on other forms such as being a mechanical feature that is felt by the user in a tactile manner, a scented feature that upon activation emits a scent, a display screen that displays various texts, or the like.

In some embodiments, the indicator 136 may light up in different colors depending on the status of the toothbrush 100 or sensor 133. For example, the indicator 136 may illuminate as blue when the sensor 133 is operably coupled to a portable electronic device as described in more detail below, the indicator 136 may be red when the toothbrush 100 or power source thereof is charging, and it may be green when the toothbrush 100 is being used in a toothbrushing session. In other embodiments, the indicator 136 may light up as red (or orange or any other color) when blood is determined to be present in the toothpaste slurry/oral cavity as described herein.

In some embodiments, there may be a button or other type of actuator (slide switch, button, capacitive sensor, etc.) on the toothbrush 100 for activating the sensor 133. Thus, prior to a toothbrushing session, a user may press (or otherwise actuate) the button to activate the sensor 133. Pressing the button (or otherwise actuating the actuator) may also activate the motor 137 when the motor 137 is included such as when the toothbrush 100 is a powered toothbrush. Alternatively, there may be separate buttons/actuators for the motor 137 and for the sensor 133. In some embodiments, there is no motor and the toothbrush 100 is a manual toothbrush.

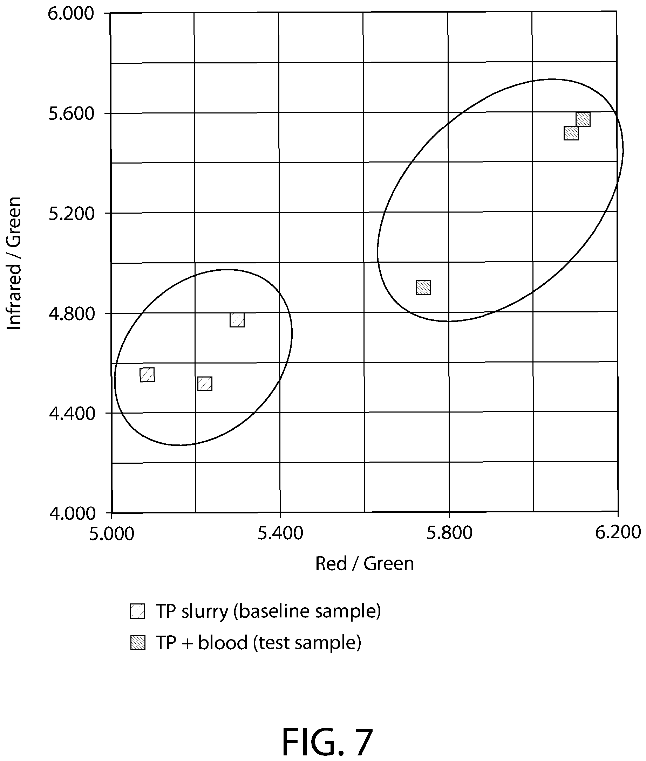

Referring to FIG. 7, a graphical representation of the results of an experimental test performed using a prototype device comprising the sensor 133 and the processor 131 is provided. To demonstrate the capability of the toothbrush 100, two samples were measured. About 2 grams of toothpaste were mixed with about 5 mL of saliva to create toothpaste / saliva slurry, this is the "baseline sample." The same procedure was repeated, and 1 drop of human blood was added to the slurry then mixed well to create the "test sample" - toothpaste / saliva slurry with blood. One drop of the baseline sample was deposited on a glass slide and the sensor 133 was put under the slide to measure the reflected light. At least 100 data points were recorded for each of the three wavelengths of light, and the average values were used in later processing. The procedure was repeated three times to acquire baseline data for toothpaste / saliva slurry. The same procedure was repeated three times for the test sample as well.

FIG. 7 shows the results of a signal measured by the prototype device. The x-axis is the signal intensity ratio of red light over green light, while the y-axis is the signal intensity ratio of infrared light over green light. Clear separation of the "baseline sample" and the "test sample" was observed. To identify and quantify hemoglobin in toothpaste slurry, different data processing algorithms can be employed, such as KNN (k-Nearest Neighbor) for identification and regression for quantification.

Using this example, if the ratio of the intensity of the reflected portion of the first (i.e., red) light to the intensity of the reflected portion of the second (i.e., green) light is greater than 5.6, it can be determined that there is hemoglobin/blood in the oral cavity (or in the toothpaste slurry and hence also in the oral cavity). Similarly, if the ratio of the intensity of the reflected portion of the third (i.e., infrared) light to the intensity of the reflected portion of the second (i.e., green) light is greater than 4.8, it can be determined that there is hemoglobin/blood in the oral cavity (or in the toothpaste slurry and hence also in the oral cavity).

In some embodiments, the sensor 133 is configured to collect data continuously once activated. For example, once powered on, the sensor 133 may collect data for a predetermined period of time, for example for 120 seconds, or 130 seconds, or 140 seconds, or 150 seconds to ensure that it is collecting data for the duration of a toothbrushing session (which is ideally 120 seconds, although more frequently a shorter period of time). In some embodiments, the sensor 133 may collect two data points every second such that it may collect 240 data points during a two minute toothbrushing session. In other embodiments, the sensor 133 may collect data intermittently. For example, the sensor 133 may collect data at ten seconds, and then at thirty seconds, and then at one minute, and then at one minute and fifteen seconds, and then at one minute and thirty seconds, and then at one minute and forth-five seconds, and then at two minutes. The exact frequency at which the sensor 133 collects data regarding the presence or absence of hemoglobin is not to be limiting of the present invention in all embodiments.

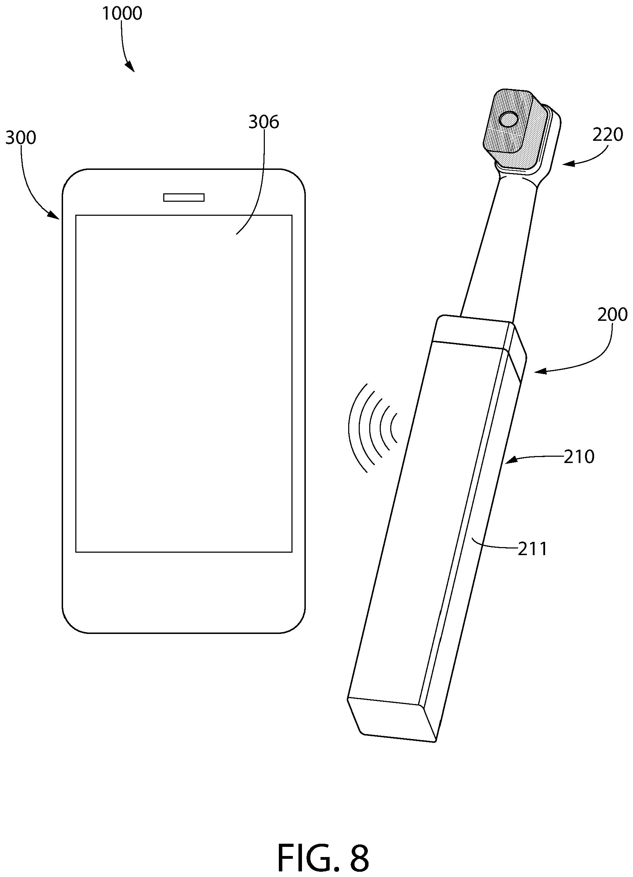

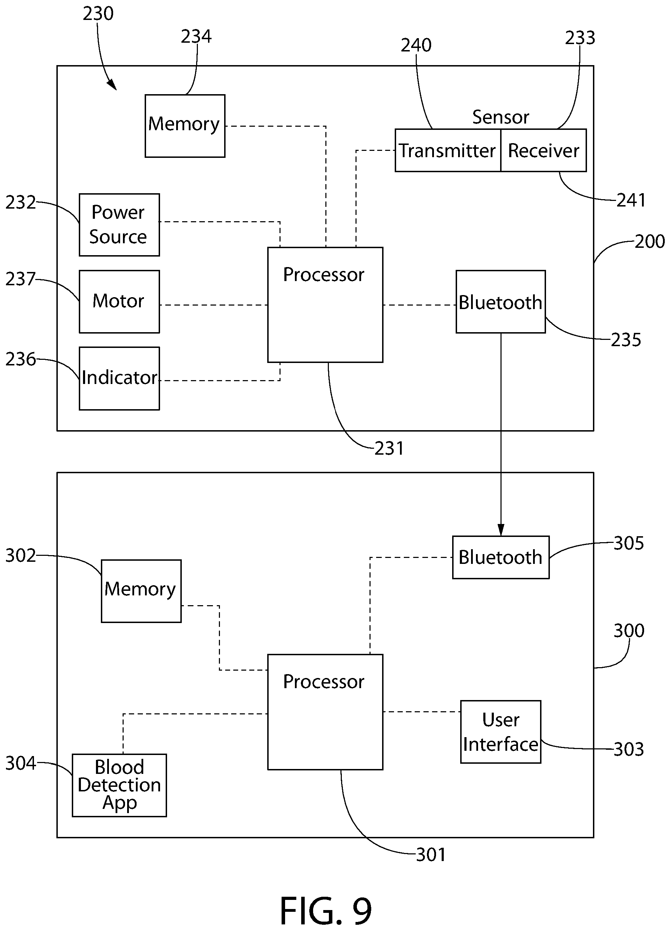

Referring to FIGS. 8 and 9, a system 1000 for detecting blood in an oral cavity during toothbrushing is illustrated. The system 1000 includes a toothbrush 200 and a portable electronic device 300 that are in operable communication with one another. The toothbrush 200 may be structurally identical to the toothbrush 100 described above with reference to FIGS. 1-3. Thus, in the exemplified embodiment the toothbrush 200 comprises a body 210 having a handle portion 211 and a stem (not illustrated) and a refill head 220 coupled to the body 210 in a detachable manner. For further details of the structure of the toothbrush 200, reference can be made to the toothbrush 100 described above.

Similar to the toothbrush 100, the toothbrush 200 comprises an electronic circuit 230 that may include a processor 231, a power source 232, a sensor 233, a memory device 234 (which could alternatively be a part of the processor 231 in some embodiments), a Bluetooth module 235, and an indicator 236. However, it may be possible to omit the processor 231, the memory device 234, and the indicator 236 in some embodiments. Thus, in some embodiments the electronic circuit 230 of the toothbrush 200 may comprise only the sensor 233 (which includes a transmitter 240 and a receiver 241), the power source 232, and the Bluetooth module 235. The toothbrush 200 may also include a motor 237 in instances where the toothbrush 200 is a powered toothbrush.

The reason that the toothbrush 200 need not include the processor 231, the memory device 234, and the indicator 236 (although it may include any of one or more of these components in some embodiments) is because these components are included as a part of the portable electronic device 300 that is in communication with the toothbrush 200. Specifically, the portable electronic device 300 may be a smart phone, a tablet, a computer, or a similar device that includes a processor 301, a memory 302, a user interface 303, a blood detection software application 304, and a Bluetooth module 305. The portable electronic device 300 may also include a display 306 (which can be the same as the user interface 303 or distinct from the user interface 303). In the exemplified embodiment, the processor 301 of the portable electronic device 300 is in operable communication with the sensor 233 of the toothbrush 200 via Bluetooth due to the incorporation of the Bluetooth module 235 in the toothbrush 200 and the Bluetooth module 305 in the portable electronic device 300 (when the toothbrush 200 and the portable electronic device 300 are in sufficiently close proximity to one another so as to allow for such a Bluetooth connection). Of course, Bluetooth is merely one exemplary way that the toothbrush 200 and the portable electronic device 300 can be in communication. In other embodiments, there may be a wired connection between the toothbrush 200 and the portable electronic device 300 or they may communicate using other wireless protocols (infrared wireless communication, satellite communication, radio, microwave, Zigbee, Z-wave, or the like). Of course, the sensor 233 may have a built-in microcontroller in some embodiments.

In this embodiment, the sensor 233 will operate in the same way as the sensor 133 described above. Thus, the transmitter 240 of the sensor 233 comprises multiple light sources that emit light at different wavelengths and the receiver 241 of the sensor 233 receives reflected light. The sensor 233 then generates signals indicative of the intensities of the various reflected lights. However, in this embodiment the signals are then transmitted, via Bluetooth or otherwise, from the sensor 233 of the toothbrush 200 to the processor 301 of the portable electronic device 300. The transmission of these signals from the sensor 233 of the toothbrush 200 to the processor 301 of the portable electronic device 300 may occur so long as the toothbrush 200 and the portable electronic device 300 are in operable communication (either via Bluetooth or other wireless technologies or through a wired connection).

In some embodiments, the information associated with the signals and the information detected by the sensor 133 may be stored in the memory 234 and/or processor 231 of the toothbrush 200 initially and then transferred to the processor 301 of the portable electronic device 300 in batches. Thus, data or information corresponding to a plurality of different toothbrushing sessions may initially be stored in the memory 234 and/or processor of the toothbrush 231. This can be useful in instances in which the user brushes his/her teeth at a time that the toothbrush 200 is not in operable communication with the portable electronic device 300. In this way, the toothbrush 200 will initially store all of the data, and then once the toothbrush 200 becomes operably coupled to the portable electronic device 300, the data can be transmitted to the portable electronic device 300 for further processing as described herein (either automatically or in response to manual user input). In such embodiments, the processor 231 may be able to process the data just like the processor 131 of the toothbrush 100 so that the processor 231 can activate the indicator 236 when it determines that hemoglobin/blood is present in the oral cavity or toothpaste slurry.

As noted above, the portable electronic device 300 may have a blood detection software application 304 downloaded thereon. Thus, during or just prior to a toothbrushing session, a user may open the blood detection software application 304 and put the toothbrush 200 into operable (wireless or wired) communication with the portable electronic device 300. Alternatively, the operable coupling between the toothbrush 200 and the portable electronic device 300 may cause the blood detection software application 304 to automatically launch on the portable electronic device 300. In such a situation, as the sensor 233 of the toothbrush 200 is gathering information/data related to the intensity of the reflected light, it will transmit this data/information to the processor 301 of the portable electronic device 300. In some embodiments, this transmission of data/information from the sensor 233 to the processor 301 may occur automatically so long as the toothbrush 200 and the portable electronic device 300 are in operable communication with one another. Of course, as noted above, this data/information can alternatively (or additionally) be stored locally on the memory device 234 of the toothbrush 200 and then transmitted to the portable electronic device 300 in batches. The processor 301 of the portable electronic device 300 may make this data/information available to a user in various ways on the portable electronic device 300 using the blood detection software application 304.

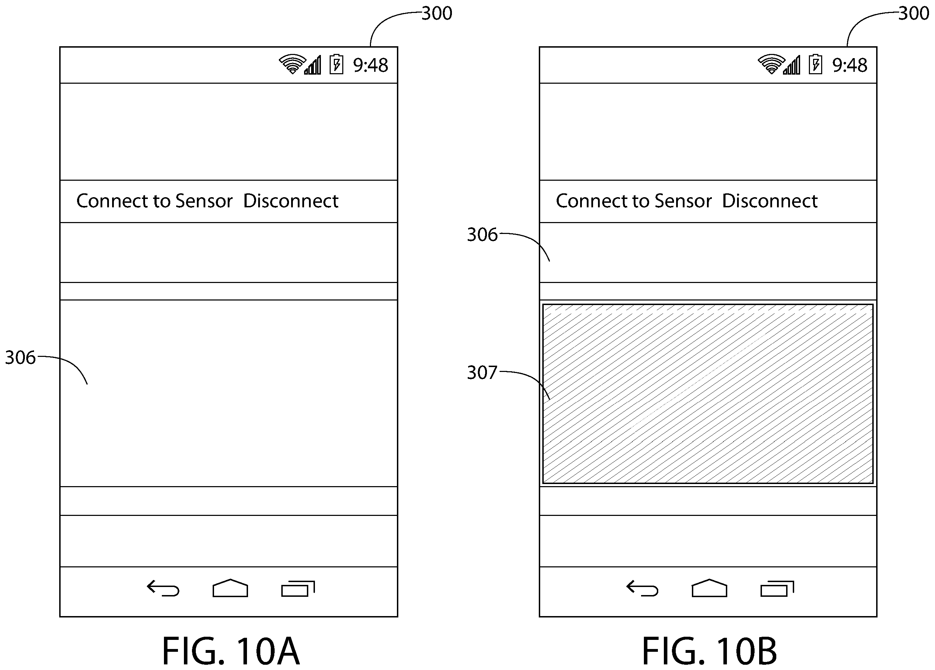

Specifically, referring first to FIGS. 10A and 10B, one embodiment of the blood detection software application 304 is illustrated as displayed on the display 306 of the portable electronic device 300. In this reasonably simple embodiment of the blood detection software application 304, the blood detection software application 304 merely informs the user whether or not blood was detected in the oral cavity (or in the toothpaste slurry) during a toothbrushing session. Thus, as the processor 301 receives the signals from the sensor 233 and processes the signals in accordance with the algorithm(s) as described herein, the processor 301 causes the blood detection software application 304 to indicate whether or not blood is present. In FIG. 10A, no blood is present and thus the display 306 on the portable electronic device 300 is blank. In FIG. 10B, blood is present and thus the display 306 on the portable electronic device 300 is depicted with a shaded area 307. This shaded area 307 may be colored red as an indication that blood is present, although other colors, designs, or the like could be used, including text being displayed to indicate whether or not blood was present. Thus, in this embodiment the shaded area 307 on the display 306 of the portable electronic device 300 serves as an indicator to a user to let the user know whether or not blood was present. Stated another way, the shaded area 307 is an output of the system 1000 that serves as an indication to a user of the presence or absence of blood in the toothpaste slurry. The processor 301 may further track this information over multiple uses in the blood detection software application 304 so that a user can review a log of data for each day indicating whether blood was present during toothbrushing or not during the toothbrushing session(s) that occurred in a given day.

In some embodiments, if blood is detected at any time during the toothbrushing session, the display 306 on the portable electronic device 300 will indicate this throughout the entirety of the toothbrushing session. In some embodiments, the display 306 on the portable electronic device 300 will update during the toothbrushing session depending on whether blood is being detected at a given time during the toothbrushing session. In some embodiments, the output on the display 306 may be an indication that blood was detected, and/or an indication of the quantity of blood detected, and/or an indication as to whether blood was detected and if so the time during the toothbrushing session that it was detected (for example, the display 306 may indicate that blood was first detected eighteen seconds into the toothbrushing session).

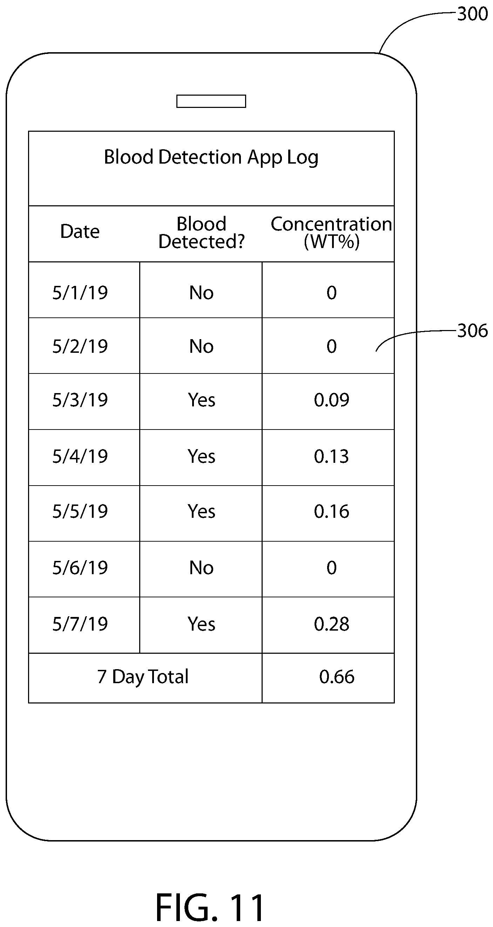

For example, referring to FIG. 11, the display 306 of the portable electronic device 300 is depicted in accordance with one embodiment with the blood detection software application 304 open so that a log of data from previous toothbrushing sessions is displayed. Thus, a user can open the blood detection software application 304 and navigate the application to the blood detection application log. In the exemplified embodiment, the log includes a date, a simple yes or no as to whether blood was detected on that particular date, and a concentration of blood that was detected as a weight percentage for each day. Various modifications can be made to the blood detection software application 304 to provide the user with any desired information or data about the blood that is or is not being detected during various toothbrushing sessions. In some embodiments, the blood detection software application 304 may be programmed with an algorithm the analyzes the sensor data log, and provides users a warning signal if continuous bleeding is detected, e.g. bleeding 7 days in a row. Thus, the depiction in FIG. 11 is merely exemplary and is not intended to illustrate the full scope of possibilities available through the blood detection software application 304. The information provided in this format may be the highest quantity of blood detected by the sensors 133, 233 during the toothbrushing session, an average of the amount of blood detected by the sensors 133, 233 during the toothbrushing session, or the like.

Thus, with the data provided in FIG. 11, a user can be informed regarding how many times they were bleeding during toothbrushing over the last week, over the last two weeks, over the last three weeks, over the last month, and so on (this timeframe can be an adjustable setting in some embodiments). Typically, when a person bleeds during toothbrushing they notice it when the expectorate the toothpaste slurry or they taste it during brushing, but they forget about the fact that they were bleeding soon after they finish toothbrushing. Thus, even if a person bleeds daily, they do not put too much thought into it. By providing the user with a log of information from past toothbrushing sessions, this can make the user more aware of the frequency of bleeding during toothbrushing so that the user can seek treatment if necessary. The data provided in FIG. 11 could be displayed to the user, for example on the display of the portable electronic device 300 or elsewhere (i.e., on a computer or wherever it may be displayed) in tabulated form, such as in a bar graph, a line graph, or the like.

In some embodiments, sensor data can be uploaded to a remote cloud server(s) for achieving further analysis. The toothbrush 100, 200 could include a WiFi chip so that it can transmit data to the cloud or to a remote server, or the toothbrush 100, 200 can transmit the data to the portable electronic device 300, which in turn can send the data to the cloud/server. Users can use their computers (larger screen) to access their long term data log to see the trend (on a software app or program, on a website, or the like), and choose to share these data with their oral care service providers for better care. Oral care service providers can review these data to monitor their patients in between their regularly scheduled visits. Researchers can utilize these data for oral health study or efficacy evaluation of existing or experimental oral care products or regimens, as well as epidemiology studies in oral care. Insurance companies can reduce their cost by requesting high risk population to take early or preventive treatments.

In some embodiments, the system 1000 or toothbrush 100 may be able to detect how much blood (i.e., quantity) that a user bleeds in a single toothbrushing session. However, this information may not be as helpful as it seems because it may be dependent on when the user brushes a particularly blood prone region of the oral cavity during the toothbrushing session. Thus, if the user tends to bleed from the gums above the first molar in the upper left quadrant of the mouth, if the user brushes the upper left quadrant of the mouth first during the toothbrushing session than there would be more blood during the toothbrushing session than if the user brushes the upper left quadrant of the mouth last during the toothbrushing session. That said, obtaining a value for the quantity of blood bled during a toothbrushing session may still have some value to a user or medical professional.

Referring again to FIG. 1, in some embodiments, the toothbrush 100 (or the toothbrush 200) may be configured to track the location in the mouth during a toothbrushing session in addition to tracking blood/hemoglobin in the oral cavity or toothpaste slurry. Thus, the toothbrush 100 may include one or more location tracking sensors (or position sensors) 139 that are configured to track the location of the toothbrush 100, 200 in the oral cavity. In the exemplified embodiment, the tracking sensor 139 is located within the stem 112 of the body 110, but it may be located anywhere so long as it is configured to operate as described herein. The tracking sensor 139 is operably coupled to the processor 131 so that the processor 131 can receive signals detected/generated by the tracking sensor 139 and process those signals to determine where in the mouth the head 120 of the toothbrush 100 is located at a given time during a toothbrushing session. For example, in some embodiments the location sensor 139 could be located in the handle portion 111 of the body 110.

One example of such a toothbrush that is configured to track the location in the oral cavity is described in United States Patent No. 10,349,733, issued on July 16, 2019. Thus, the one or more location tracking sensors may be accelerometers, motion sensors, intertial sensors, gyroscopes, magnetometers, and other sensors capable of detecting positions, movement, and acceleration. The location tracking sensors may transmit signals to the processor 131, 231 so that the processor 131, 231 may be configured to determine where in the oral cavity the toothbrush head is located at a given time during the toothbrushing session. The tracking sensor 139 may be a single sensor or it may be multiple sensors, and it may comprise sensors of different types (accelerometers, gyroscopes, proximity sensors, etc.).

Thus, combining this location tracking with the blood tracking, the processor 131, 231 may keep a log of where in the mouth the toothbrush is located when blood is first detected. Because the sensors 133, 233 may take measurements/collect data every second in some embodiments, the sensors 133, 233 will detect blood almost instantaneously. Thus, if the head of the toothbrush 100, 200 is located in the upper right quadrant of the mouth and the gums in that region start to bleed, the system 1000 will be able to track this information and provide it to the user (such as via the blood detection software application 304 or the like). Specifically, the system 1000 will know where the toothbrush 100, 200 was located at the time that blood was first detected, which is a good indication that the blood is coming from the region of the oral cavity that the toothbrush 100, 200 is located at that time. The system 1000 may be able to track locations of bleeding based on which of four quadrants (upper left, upper right, lower left, lower right) of the oral cavity that the bleeding occurs or it may be able to provide more specific information such as that the gums above the first molar in the upper left quadrant of the mouth are bleeding. Furthermore, it need not be based on four quadrants and could instead simply track whether the blood is coming from the top or bottom of the oral cavity. In still other embodiments, the oral cavity may be divided into more than four quadrants to provide a more precise indication as to where the blood is coming from. In this way, the system 1000 (or toothbrush 100) will be able to track which part of the oral cavity is bleeding and provide that information to the user or to a medical professional. This can be beneficial information for a user to provide to a medical professional or simply for the user to have so that the user can treat that area of the oral cavity as needed.

Thus, the system 1000 (or the toothbrushes 100, 200) may determine that at forty-five seconds into the toothbrushing session, blood was first detected. The system 1000 can then determine where in the mouth/oral cavity the toothbrush 100, 200 (or the head or cleaning elements thereof) was located forty-five seconds into the toothbrushing session. In this way, the system 1000 can determine the location within the oral cavity of the toothbrush 100, 200 at the time that blood was first detected, which is very likely to be the location of the oral cavity that is bleeding. This information can be provided to the user on a graphical display. For example, the display 306 on the portable electronic device 300 may show a visual representation of a set of teeth or an oral cavity, and the display may indicate which region of the oral cavity (such as by highlighting that region or coloring it red or the like) the blood was first detected.

Along these same lines, the system 1000 (or the toothbrush 100, 200 itself) may also be able to determine when there is a second location within the oral cavity that is bleeding. For example, the system 1000 may keep track of the quantity/amount of blood that is in the oral cavity during the toothbrushing session. If at any time there is a significant increase in the amount of blood detected, the system 1000 may determine that there is a second location in the oral cavity that is bleeding. Thus, for example, if it is detected ten seconds into the toothbrushing session that there is 0.1ml of blood and then at forty-five seconds into the toothbrushing session it is determined that there is 0.3ml of blood, then the system 1000 may understand this to mean that there is a second location that is bleeding. Thus, the system 1000 may determine the location of the head of the toothbrush at the moment that the quantity of blood increased to determine the second bleeding location in the oral cavity.

Operation of the system 1000 will now be briefly described in accordance with a method of detecting blood in a toothpaste slurry during toothbrushing. The method includes having a user brush his or her teeth and gums with the cleaning elements 123 of the toothbrush 100 during a toothbrushing session. During such toothbrushing, if toothpaste has been pre-applied onto the cleaning elements 123, a toothpaste slurry will be formed in the oral cavity during the toothbrushing session. Next, signals containing information related to the presence or absence of hemoglobin in the toothpaste slurry is generated. In accordance with the exemplified embodiment, these signals are generated with a sensor 133 that is coupled to the toothbrush 100. More specifically, the sensor 133 will transmit first light at a first wavelength and second light at a second wavelength into the oral cavity where the toothpaste slurry is located. Portions of the first and second light will then be reflected off of the toothpaste slurry. A receiver 141 of the sensor 133 will receive the reflected portions of the first and second light.

Next, the processor 131, 301 will receive the signals corresponding to the intensities of the reflected portions of the first and second light. The processor 131, 301 will process these signals to determine whether hemoglobin (and therefore blood) is present in the toothpaste slurry. This may be achieved by the processor calculating a ratio of the first intensity of the reflected portion of the first light to the second intensity of the reflected portion of the second light. Using the results of this calculation, the processor 131, 301 can determine whether hemoglobin/blood is present in the toothpaste slurry. In some instances, when the processor 131, 301 determines that hemoglobin is present in the toothpaste slurry, the method may include providing an indication of the presence of blood in the toothpaste slurry to a user. This may involve displaying such an indication on the display 306 of the portable electronic device 300 or activating an indicator 136 located on the toothbrush 100.

The above described system and method is a "reagent" free system and method to measure hemoglobin using only a few wavelengths of light (2-3) in both visible and infrared regions during tooth brushing. The surfactant Sodium Lauryl Sulfate ("SLS") in most toothpaste compositions serves as the stabilizing agent for hemoglobin, so the method does not require additional reagent specific for hemoglobin detection.

It should be noted that although the description provided herein relates to using the toothbrush 100, 200 and/or system 1000 to detect hemoglobin in order to determine whether there is blood in the oral cavity and/or toothpaste slurry, the invention is not to be so limited in all embodiments. For example, in some embodiments the toothbrush 100, 200 and/or system 1000 may be used to detect Serum albumin, which is also abundant in blood. However, Serum albumin does not have a strong spectral signature. Thus, when the toothbrush 100, 200 and/or system 1000 is used to detect Serum albumin, a dye such as bromocresol green or the like may first be added. The Serum albumin with a dye as noted herein would absorb red light, so the sensor 133, 233 noted herein could be used to detect this Serum albumin/dye in the same manner as noted above in order to determine whether blood is present, although the algorithm would have to be modified so that red light is the denominator in the equations.

## Claims

1. A system (1000) for detecting blood in an oral cavity during toothbrushing, the system comprising:
a toothbrush (200) comprising an electronic circuit (230) comprising a sensor (233) that is configured to generate signals related to the presence or absence of hemoglobin in the oral cavity during a toothbrushing session; and
a portable electronic device (300) comprising a processor (301) that is operably coupled to the sensor (233) of the toothbrush (200), the processor (301) configured to receive and process the signals to determine whether hemoglobin is present in the oral cavity during the toothbrushing session,
wherein the sensor (233) is configured to emit first light (145) at a first wavelength and second light (146) at a second wavelength, receive reflected portions of the first light (145) and the second light (146), and generate a first signal indicative of a first intensity of the reflected portion of the first light (145) and a second signal indicative of a second intensity of the reflected portion of the second light (146);
wherein the processor (301) is configured to calculate a ratio of the first intensity to the second intensity to determine whether hemoglobin is present in the oral cavity during the toothbrushing session; and
wherein the first light (145) is red light or infrared light and the second light (146) is green light.

2. The system (1000) according to claim 1 wherein the electronic circuit (230) of the toothbrush (200) further comprises a power source (232) operably coupled to the sensor (233) for supplying power thereto.

3. The system (1000) according to claim 1 or claim 2 further comprising a software application (304) stored on the portable electronic device (300), wherein the software application (304) comprises an indicator (307) that is activated when the processor (301) determines that hemoglobin is present in the oral cavity during the toothbrushing session; wherein the indicator (307) is a visual indicator that is displayed on a display screen (306) of the portable electronic device (300); and wherein the software application (304) stores information related to the presence of hemoglobin in the oral cavity for each of a plurality of distinct toothbrushing sessions, and wherein the information is displayed on the display screen (306) of the portable electronic device (300).

4. The system (1000) according to any one of claims 1 to 3 wherein the processor (301) of the portable electronic device (300) is operably coupled to the sensor (233) of the toothbrush (200) via Bluetooth.

5. A system (1000) for detecting blood in a toothpaste slurry (150, 151) during a toothbrushing session, the system (1000) comprising:
a sensor (133) configured to generate signals related to the presence or absence of hemoglobin in the toothpaste slurry (150, 151) during the toothbrushing session;
a processor (131) operably coupled to the sensor (133) and configured to receive and process the signals to determine whether hemoglobin is present in the toothpaste slurry (150, 151) during the toothbrushing session; and
an output (136) configured to inform a user whether blood is present in the toothpaste slurry (150, 151) during the toothbrushing session based on the processing performed by the processor (131),
wherein the sensor (133) is configured to emit first light (145) at a first wavelength and second light (146) at a second wavelength, receive reflected portions of the first light (145) and the second light (146) that are reflected from the toothpaste slurry (150, 151), and generate a first signal indicative of a first intensity of the reflected portion of the first light (145) and a second signal indicative of a second intensity of the reflected portion of the second light (146), and
wherein the processor (131) is configured to calculate a ratio of the first intensity to the second intensity to determine whether hemoglobin is present in the toothpaste slurry (150, 151).

6. The system (1000) according to claim 5 further comprising a toothbrush (200) having cleaning elements (123), the toothbrush (200) comprising the sensor (133); and a portable electronic device (300) comprising the processor (131), the portable electronic device (300) being in operable communication with the toothbrush (200); wherein the toothbrush (200) comprises the processor (131) and wherein the output (136) comprises activating an indicator (136) on the toothbrush (200).

## Patentansprüche

1. System (1000) zum Erkennen von Blut in einer Mundhöhle während des Zähneputzens, wobei das System umfasst:
eine Zahnbürste (200) mit einer elektronischen Schaltung (230), die einen Sensor (233) umfasst, der so konfiguriert ist, dass er Signale erzeugt, die sich auf das Vorhandensein oder Nichtvorhandensein von Hämoglobin in der Mundhöhle während einer Zahnputzsitzung beziehen; und
eine tragbare elektronische Vorrichtung (300), die einen Prozessor (301) umfasst, der funktionsfähig mit dem Sensor (233) der Zahnbürste (200) gekoppelt ist, wobei der Prozessor (301) so konfiguriert ist, dass er die Signale empfängt und verarbeitet, um zu bestimmen, ob während der Zahnputzsitzung Hämoglobin in der Mundhöhle vorhanden ist,
wobei der Sensor (233) so konfiguriert ist, dass er ein erstes Licht (145) mit einer ersten Wellenlänge und ein zweites Licht (146) mit einer zweiten Wellenlänge aussendet, reflektierte Abschnitte des ersten Lichts (145) und des zweiten Lichts (146) empfängt und ein erstes Signal erzeugt, das eine erste Intensität des reflektierten Abschnitts des ersten Lichts (145) anzeigt, sowie ein zweites Signal erzeugt, das eine zweite Intensität des reflektierten Abschnitts des zweiten Lichts (146) anzeigt;
wobei der Prozessor (301) so konfiguriert ist, dass er ein Verhältnis der ersten Intensität zur zweiten Intensität berechnet, um zu bestimmen, ob während der Zahnputzsitzung Hämoglobin in der Mundhöhle vorhanden ist; und
wobei das erste Licht (145) rotes Licht oder Infrarotlicht ist und das zweite Licht (146) grünes Licht ist.

2. System (1000) nach Anspruch 1, wobei die elektronische Schaltung (230) der Zahnbürste (200) ferner eine Energieversorgung (232) umfasst, die funktionsfähig mit dem Sensor (233) gekoppelt ist, um diesen mit Strom zu versorgen.

3. System (1000) nach Anspruch 1 oder Anspruch 2, das ferner eine auf der tragbaren elektronischen Vorrichtung (300) gespeicherte Softwareanwendung (304) umfasst, wobei die Softwareanwendung (304) einen Indikator (307) umfasst, der aktiviert wird, wenn der Prozessor (301) bestimmt, dass während der Zahnputzsitzung Hämoglobin in der Mundhöhle vorhanden ist; wobei der Indikator (307) ein visueller Indikator ist, der auf einem Bildschirm (306) der tragbaren elektronischen Vorrichtung (300) angezeigt wird; und wobei die Softwareanwendung (304) Informationen über das Vorhandensein von Hämoglobin in der Mundhöhle für jede einer Mehrzahl von unterschiedlichen Zahnputzsitzungen speichert und wobei die Informationen auf dem Bildschirm (306) der tragbaren elektronischen Vorrichtung (300) angezeigt werden.

4. System (1000) nach Anspruch 1 bis 3, wobei der Prozessor (301) der tragbaren elektronischen Vorrichtung (300) über Bluetooth funktionsfähig mit dem Sensor (233) der Zahnbürste (200) gekoppelt ist.

5. System (1000) zum Erkennen von Blut in einer Zahnpastasuspension (150, 151) während einer Zahnputzsitzung, wobei das System (1000) umfasst:
einen Sensor (133), der so konfiguriert ist, dass er Signale erzeugt, die sich auf das Vorhandensein oder Nichtvorhandensein von Hämoglobin in der Zahnpastasuspension (150, 151) während der Zahnputzsitzung beziehen;
einen Prozessor (131), der funktionsfähig mit dem Sensor (133) gekoppelt ist und so konfiguriert ist, dass er die Signale empfängt und verarbeitet, um zu bestimmen, ob während der Zahnputzsitzung Hämoglobin in der Zahnpastasuspension (150, 151) vorhanden ist; und
eine Ausgabe (136), die so konfiguriert ist, dass sie einem Benutzer mitteilt, ob während der Zahnputzsitzung Blut in der Zahnpastasuspension (150, 151) vorhanden ist, auf der Grundlage der vom Prozessor (131) durchgeführten Verarbeitung,
wobei der Sensor (133) so konfiguriert ist, dass er ein erstes Licht (145) mit einer ersten Wellenlänge und ein zweites Licht (146) mit einer zweiten Wellenlänge aussendet, reflektierte Abschnitte des ersten Lichts (145) und des zweiten Lichts (146), die von der Zahnpastasuspension (150, 151) reflektiert werden, empfängt und ein erstes Signal erzeugt, das eine erste Intensität des reflektierten Abschnitts des ersten Lichts (145) anzeigt, sowie ein zweites Signal erzeugt, das eine zweite Intensität des reflektierten Abschnitts des zweiten Lichts (146) anzeigt, und
wobei der Prozessor (131) so konfiguriert ist, dass er ein Verhältnis der ersten Intensität zur zweiten Intensität berechnet, um zu bestimmen, ob Hämoglobin in der Zahnpastasuspension (150, 151) vorhanden ist.

6. System (1000) nach Anspruch 5, das ferner eine Zahnbürste (200) mit Reinigungselementen (123) umfasst, wobei die Zahnbürste (200) den Sensor (133) umfasst; und eine tragbare elektronische Vorrichtung (300), die den Prozessor (131) umfasst, wobei die tragbare elektronische Vorrichtung (300) in funktionsfähiger Kommunikation mit der Zahnbürste (200) steht; wobei die Zahnbürste (200) den Prozessor (131) umfasst und wobei die Ausgabe (136) ein Aktivieren einer Anzeige (136) auf der Zahnbürste (200) umfasst.

## Revendications

1. Système (1000) de détection de sang dans une cavité buccale lors du brossage des dents, le système comprenant:
une brosse à dents (200) comprenant un circuit électronique (230) comprenant un capteur (233) conçu pour générer des signaux liés à la présence ou à l'absence d'hémoglobine dans la cavité buccale lors d'une session de brossage des dents; et
un dispositif électronique portable (300) comprenant un processeur (301) couplé de manière fonctionnelle au capteur (233) de la brosse à dents (200), le processeur (301) étant conçu pour recevoir et traiter les signaux afin de déterminer si de l'hémoglobine est présente dans la cavité buccale pendant la session de brossage des dents,
dans lequel le capteur (233) est conçu pour émettre une première lumière (145) à une première longueur d'onde et une seconde lumière (146) à une seconde longueur d'onde, recevoir des parties réfléchies de la première lumière (145) et de la seconde lumière (146), et générer un premier signal indiquant une première intensité de la fraction réfléchie de la première lumière (145) et un second signal indiquant une seconde intensité de la fraction réfléchie de la seconde lumière (146);
dans lequel le processeur (301) est conçu pour calculer un rapport de la première intensité à la seconde intensité afin de déterminer si de l'hémoglobine est présente dans la cavité buccale pendant la session de brossage des dents; et
dans lequel la première lumière (145) est une lumière rouge ou une lumière infrarouge et la seconde lumière (146) est une lumière verte.

2. Système (1000) selon la revendication 1, dans lequel le circuit électronique (230) de la brosse à dents (200) comprend en outre une source d'alimentation (232) couplée de manière fonctionnelle au capteur (233) pour lui fournir de l'énergie.

3. Système (1000) selon la revendication 1 ou la revendication 2, comprenant en outre une application logicielle (304) stockée sur le dispositif électronique portable (300), dans lequel l'application logicielle (304) comprend un indicateur (307) qui est activé lorsque le processeur (301) détermine que de l'hémoglobine est présente dans la cavité buccale pendant la session de brossage des dents; dans lequel l'indicateur (307) est un indicateur visuel qui est affiché sur un écran d'affichage (306) du dispositif électronique portable (300); et dans lequel l'application logicielle (304) stocke des informations relatives à la présence d'hémoglobine dans la cavité buccale pour chacune d'une pluralité de sessions de brossage des dents distinctes, et dans lequel les informations sont affichées sur l'écran d'affichage (306) du dispositif électronique portable (300).

4. Système (1000) selon l'une quelconque des revendications 1 à 3, dans lequel le processeur (301) du dispositif électronique portable (300) est couplé de manière fonctionnelle au capteur (233) de la brosse à dents (200) via Bluetooth.

5. Système (1000) de détection de sang dans une bouillie de pâte dentifrice (150, 151) lors d'une session de brossage des dents, le système (1000) comprenant:
un capteur (133) conçu pour générer des signaux liés à la présence ou à l'absence d'hémoglobine dans la bouillie de pâte dentifrice (150, 151) pendant la session de brossage des dents;
un processeur (131) couplé de manière fonctionnelle au capteur (133) et conçu pour recevoir et traiter les signaux afin de déterminer si de l'hémoglobine est présente dans la bouillie de pâte dentifrice (150, 151) pendant la session de brossage des dents; et
une sortie (136) conçue pour informer un utilisateur si du sang est présent dans la bouillie de pâte dentifrice (150, 151) pendant la session de brossage des dents sur la base du traitement effectué par le processeur (131),
dans lequel le capteur (133) est conçu pour émettre une première lumière (145) à une première longueur d'onde et une seconde lumière (146) à une seconde longueur d'onde, recevoir des parties réfléchies de la première lumière (145) et de la seconde lumière (146) qui sont réfléchies par la bouillie de pâte dentifrice (150, 151), et générer un premier signal indiquant une première intensité de la fraction réfléchie de la première lumière (145) et un second signal indiquant une seconde intensité de la fraction réfléchie de la seconde lumière (146), et
dans lequel le processeur (131) est conçu pour calculer un rapport de la première intensité à la seconde intensité afin de déterminer si de l'hémoglobine est présente dans la bouillie de pâte dentifrice (150, 151).

6. Système (1000) selon la revendication 5, comprenant en outre une brosse à dents (200) comportant des éléments de nettoyage (123), la brosse à dents (200) comprenant le capteur (133); et un dispositif électronique portable (300) comprenant le processeur (131), le dispositif électronique portable (300) étant en communication fonctionnelle avec la brosse à dents (200); dans lequel la brosse à dents (200) comprend le processeur (131) et dans lequel la sortie (136) comprend l'activation d'un indicateur (136) sur la brosse à dents (200).
